# EUROPEAN PATENT APPLICATION

(11) **EP 3 382 034 A1**
(43) Date of publication of application: **03.10.2018**
(21) Application number: 17164230.9
(22) Date of filing: 31.03.2017
(51) Int. Cl.: C12Q 1/68

(54) **GENE EXPRESSION ANALYSIS BY MEANS OF GENERATING A CIRCULARIZED SINGLE STRANDED CDNA LIBRARY**

(71) Applicant: Rheinische Friedrich-Wilhelms-Universität Bonn, 53113 Bonn (DE)
(72) Inventor: HORNUNG, Veit, 81245 München (DE); SCHMID-BURGK, Jonathan, 53113 Bonn (DE)
(74) Representative: Viering, Jentschura & Partner mbB Patent- und Rechtsanwälte

(57) **Abstract**

The present invention is directed to methods and kits for analysis of nucleic acid samples. The methods of the invention comprise the steps of a) providing nucleic acid; b) transcribing the nucleic acid to form single-stranded DNA having at its 3'-end a ddATP, ddCTP, ddGTP, or ddTTP nucleotide or a dATP, dCTP, dGTP, or dTTP nucleotide comprising a modified 3'-moiety by contacting the nucleic acid with a DNA polymerase, a primer and a mixture of (a) dNTPs and (b) at least one ddNTP or at least one dNTP comprising the modified 3'-moiety under conditions that allow the generation of the DNA, wherein the primer comprises a target-complementary region and optionally wherein the amounts of dNTPs are essentially equimolar; c) degrading the non-elongated primer; d) optionally purifying the DNA; e) contacting the DNA with a 3' to 5' exonuclease to remove the ddATP, ddCTP, ddGTP or ddTTP nucleotide or the dATP, dCTP, dGTP, or dTTP nucleotide comprising the modified 3'-moiety; f)i) contacting the DNA with a ssDNA ligase to circularize the DNA; or f)ii) contacting the DNA with (1) a linker molecule comprising a nucleic acid portion; and (2) a DNA ligase to ligate the DNA to the linker molecule; g) optionally amplifying the DNA; h) optionally quantifying the DNA by qPCR; and i) sequencing the DNA. The kits comprise the components necessary to perform the methods of the invention.

## Description

### Field of the invention

The present invention is directed to a method and kits to analyze nucleic acid composition of a single cell or a cell population or the presence of nucleic acid in a sample. In particular, the invention relates methods for gene analysis, preferably gene expression profiling comprising: a) providing nucleic acid; b) transcribing the nucleic acid to form single-stranded DNA having at its 3'-end a ddATP, ddCTP, ddGTP, or ddTTP nucleotide or a dATP, dCTP, dGTP, or dTTP nucleotide comprising a modified 3'-moiety by contacting the nucleic acid with a DNA polymerase, a primer and a mixture of (a) dNTPs and (b) at least one ddNTP or at least one dNTP comprising the modified 3'-moiety under conditions that allow the generation of the DNA, wherein the primer comprises a target-complementary region and optionally wherein the amounts of dNTPs are essentially equimolar; c) degrading the non-elongated primer; d) optionally purifying the DNA; e) contacting the DNA with a 3' to 5' exonuclease to remove the ddATP, ddCTP, ddGTP or ddTTP nucleotide or the dATP, dCTP, dGTP, or dTTP nucleotide comprising the modified 3'-moiety; f)i) contacting the DNA with a ssDNA ligase to circularize the DNA; or f)ii) contacting the DNA with (1) a linker molecule comprising a nucleic acid portion; and (2) a DNA ligase to ligate the DNA to the linker molecule; g) optionally amplifying the DNA; h) optionally quantifying the DNA by qPCR; and i) sequencing the DNA. The kits comprise the components necessary to perform the methods of the invention.

### Background of the invention

There are various kinds of methods to determine a comprehensive expression profile of a cell or cell population or to quantify individual sequences in a nucleic acid in a complex sample, one of which is the established method of sequencing. Sequencing of nucleic acid molecules became a very important analytic technique in modern molecular biology in the recent years. The development of reliable methods for DNA sequencing has been crucial for understanding the function and control of genes and for applying many of the basic techniques of molecular biology. These methods have also become increasingly important as tools in genomic analysis and many non-research applications, such as genetic identification, forensic analysis, genetic counseling, medical diagnostics and many others.

The determination of the RNA content of a cell or a tissue via sequencing provides a method for functional analysis. In existing methodologies prior to the sequencing the expressed and isolated mRNA is reverse transcribed *in vitro* into cDNA molecules followed by random shearing into cDNA fragments. Those fragments are tagged with linker sequences that are used to specifically amplify these fragments via a PCR step. The library of PCR amplicons obtained as such can be sequenced via various sequencing processes, e.g. deep sequencing or next generation deep sequencing methods (See e.g. Ronaghi, M. (2001), Genome Research 11:3-11; Rothberg JM, et al. Nature 475(7356):348-52.; Mardis ER, Trends Genet. (2008), Vol. 24(3):133-41.; Liu L et al., J Biomed Biotechnol (2012): 251364; Henson J, et al., Pharmacogenomics (2012), 13(8):901-15; Ruan X et al., Methods Mol Biol. (2012), 809:535-62. Fullwood MJ et al., Genome Res. (2009), 19(4):521-32).

A drawback of these PCR-step-based methods is the unreliable quantification of rare DNA and mRNA/cDNA molecules, respectively. This is due to the fact that PCR amplification can introduce unevenness in coverage of individual sequences. The addition of molecular random identifiers to the generated DNA fragments at the stage of DNA synthesis or reverse transcription in case of mRNA as starting material has been shown to allow eliminating the uneven coverage bioinformatically by counting individual DNA molecules only once. However, this method relies on a high sequencing coverage, which is a cost- and time-consuming procedure.

Generally, after the DNA polymerase reaction unincorporated primers have to be removed from the sample before sequencing, since otherwise the primers will dominate the sequencing reads and thereby reduce effective sequencing coverage of cDNA molecules. Typically, this is achieved by size dependent separation of the molecules, e.g. via polyacrylamide gel electrophoresis (PAGE), which suffers from poor quantitative yield and poor discrimination between molecules of similar sizes.

The European patent application EP3015554 discloses a method for gene expression analysis using for the synthesis of cDNA a dNTP mixture that comprises dUTP. However, said method provides a low percentage of usable sequencing reads after sequencing of the cDNA as the dUTP may be incorporated in the poly-A region of the investigated nucleotide. Moreover, based on the incorporation of dUTP in the cDNA, the length of the resulting circularized DNA depends on the ratio of dTTP/dUTP and cannot be adjusted by the amount of other dNTPs comprised in the amplification mixture. As the ratio of pyrimidine nucleotides in genomic DNA can significantly vary among different species, said method may require appropriate experimental modifications to be adjusted to a nucleotide sample of a new species to provide circularized DNA having an optimal length for further sequencing. Additionally, it is pointed out that only a few polymerases or transcriptases catalyze the incorporation of dUTP, therefore the number of enzymes that can be used for the transcription step in the method of EP3015554 is limited.

Hence, there is need for methods that allow improved gene expression analysis and overcome at least some of the above-mentioned drawbacks of existing technologies.

### Summary of the invention

It is an object of the present invention to meet the above need by providing methods and kits to produce DNA libraries, which can directly be sequenced without prior amplification and which offer accurate determination of nucleic acid molecules even at low sequencing density. The inventors surprisingly found that this can be achieved by incorporation of a ddNTP or a dNTP comprising a modified 3'-moiety into the generated DNA, specific degradation of the non-elongated primers, partial digestion of the 3' terminus of the DNA, cyclization of the DNA fragments and subsequent sequencing thereof.

In one aspect, the invention is thus directed to a method for gene analysis, preferably gene expression profiling comprising:
a) providing nucleic acid;
b) transcribing the nucleic acid to form single-stranded DNA having at its 3'-end a ddATP, ddCTP, ddGTP, or ddTTP nucleotide or a dATP, dCTP, dGTP, or dTTP nucleotide comprising a modified 3'-moiety by contacting the nucleic acid with a DNA polymerase, a primer and a mixture of (a) dNTPs and (b) at least one ddNTP or at least one dNTP comprising the modified 3'-moiety under conditions that allow the generation of the DNA, wherein the primer comprises a target-complementary region and optionally wherein the amounts of dNTPs are essentially equimolar;
c) degrading the non-elongated primer;
d) optionally purifying the DNA;
e) contacting the DNA with a 3' to 5' exonuclease to remove the ddATP, ddCTP, ddGTP or ddTTP nucleotide or the dATP, dCTP, dGTP, or dTTP nucleotide comprising the modified 3'-moiety;
f)i) contacting the DNA with a ssDNA ligase to circularize the DNA; or
f)ii) contacting the DNA with
   (1) a linker molecule comprising a nucleic acid portion; and
   (2) a DNA ligase to ligate the DNA to the linker molecule;
g) optionally amplifying the DNA;
h) optionally quantifying the DNA by qPCR;
i) sequencing the DNA.

In another aspect, the present invention is directed to a kit for gene analysis, preferably gene expression profiling, comprising
(A) DNA polymerase;
(B) one or more primers comprising a target-complementary region;
(C) a composition comprising dNTPs;
(D) a composition comprising ddATP, ddCTP, ddGTP, ddTTP or a dATP, dCTP, dGTP, or dTTP nucleotide comprising a modified 3'-moiety or any combination thereof, optionally in form of a mixture with the composition of (C);
(E) 3' to 5' exonuclease not catalyzing the digestion of a nucleic acid having at its 3' terminal end a ddATP, ddCTP, ddGTP, ddTTP, or a nucleotide of dATP, dCTP, dGTP, or dTTP comprising a modified 3'-moiety, preferably Exonuclease T and/or Exonuclease I;
(F) ssDNA ligase; and
(G) 3' to 5' exonuclease catalyzing the digestion of a nucleic acid having at its 3' terminal end a ddATP, ddCTP, ddGTP, ddTTP, or a nucleotide of dATP, dCTP, dGTP, or dTTP comprising a modified 3'-moiety.

### Brief description of the drawings

The invention will be better understood with reference to the detailed description when considered in conjunction with the non-limiting examples and the accompanying drawings.

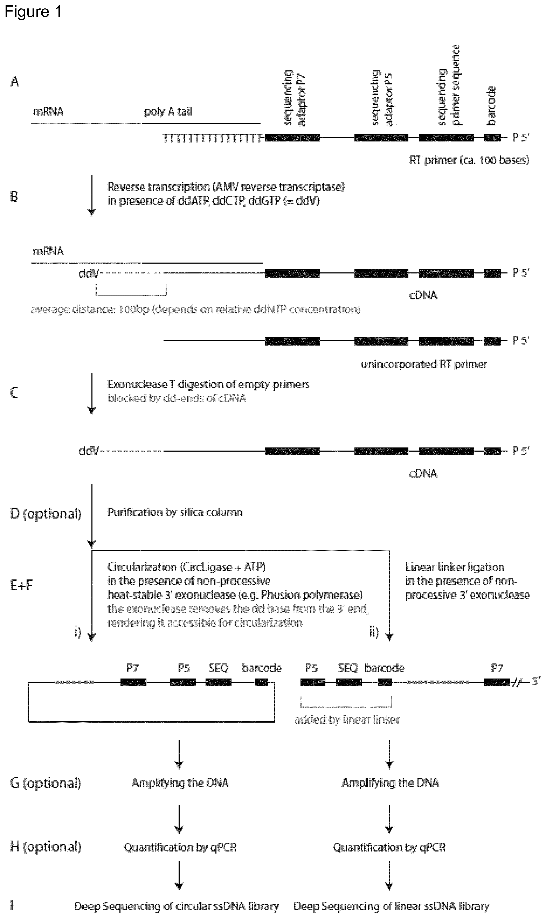

**Figure 1** schematically illustrates the steps for producing a circularized cDNA library according to the described methods. In a first step reverse transcription of isolated mRNA into cDNA using the Avian Myeloblastosis Virus (AMV) reverse transcriptase is carried out. The primer is an RT primer and is phosphorylated at its 5' end, contains deep sequencing-specific adapters, and a unique barcode sequence.
**Figure 2** shows that the cDNA average fragment length can be controlled by ddNTP concentration using AMV RT.
**Figure 3** shows that Exonuclease T degrades excess primer, but not ddNTP-terminated cDNA.
**Figure 4** shows that 3' ddNTP-blocked ssDNA can be circularized by CircLigase in presence of Phusion Polymerase.
**Figure 5** shows MiSeq sequencing result of the method of the present invention (cf. New protocol (ddNTP-based)) compared to the CircleSeq protocol of the European patent application EP3015554 (cf. old protocol (dUTP-based)).
**Figure 6** shows alignments of sequencing reads determined by the method of the present invention and the human genome. The read density is shown compared to the entire genome (Figure 6A) as well as in two selected protein-coding gene loci (Figure 6B). Data concerning the read density generated by the method present of the present invention are based on experiments using HEK 293T cells as described herein. The outer circle in Figure 6A depicts the gene density of the whole genome.

### Detailed description of the invention

The present invention is directed to a method of gene expression profiling and identification of nucleic acid in a sample.

The present inventors found that the method of the present applications provides improved accuracy of usable reads after sequencing and can be put into practice with a broad range of nucleotides and enzymes compared to methods in the art, specifically the method disclosed in European patent application EP3015554.

The present application relates to a method for gene analysis, preferably gene expression profiling comprising:
a) providing nucleic acid;
b) transcribing the nucleic acid to form single-stranded DNA having at its 3'-end a ddATP, ddCTP, ddGTP, or ddTTP nucleotide or a dATP, dCTP, dGTP, or dTTP nucleotide comprising a modified 3'-moiety by contacting the nucleic acid with a DNA polymerase, a primer and a mixture of (a) dNTPs and (b) at least one ddNTP or at least one dNTP comprising the modified 3'-moiety under conditions that allow the generation of the DNA, wherein the primer comprises a target-complementary region and optionally wherein the amounts of dNTPs are essentially equimolar;
c) degrading the non-elongated primer;
d) optionally purifying the DNA;
e) contacting the DNA with a 3' to 5' exonuclease to remove the ddATP, ddCTP, ddGTP or ddTTP nucleotide or the dATP, dCTP, dGTP, or dTTP nucleotide comprising the modified 3'-moiety;
f)i) contacting the DNA with a ssDNA ligase to circularize the DNA; or
f)ii) contacting the DNA with
   (1) a linker molecule comprising a nucleic acid portion; and
   (2) a DNA ligase to ligate the DNA to the linker molecule;
g) optionally amplifying the DNA;
h) optionally quantifying the DNA by qPCR;
i) sequencing the DNA.

"Gene analysis", as used herein, means any analysis that may be suitable for analyzing genes.

"Gene expression", as used herein, relates to a process in which information from a gene is used for the synthesis of a gene product. In cell-based expression systems the expression comprises transcription and translation steps.

The term "gene expression profiling", as used herein, refers to method(s) for determining the mRNA expression profile of a given cell or a population of cells at a given time under a given set of conditions.

The invention is based on the inventors' finding that a library of circular DNA molecules for high-throughput deep sequencing procedures can be produced by a method that employs synthesis of a single stranded DNA complementary to a nucleic acid molecule of interest by using DNA polymerase that introduces ddNTPs into the DNA molecules thereby protecting and restricting the length of the DNA, subsequent primer degradation and circularization of the processed DNA.

The term "DNA" as used herein refers to any type of DNA molecules and also includes cDNA.

The term "cDNA", as used herein, refers to single-stranded complementary DNA that is reverse transcribed from mRNA and is therefore a copy of the intron-free biologically active RNA.

The method of the invention can be performed without prior amplification and optionally also without purification after DNA synthesis reaction, typically a reverse transcription reaction.

The term "amplification" as used herein, refers to a process by which extra or multiple copies of a particular polynucleotide are formed. Amplification includes methods such as PCR, ligation amplification (or ligase chain reaction, LCR), rolling circle amplification and amplification methods.

The term "reverse transcription", means a method wherein a cDNA copy of an mRNA molecule is synthesized. By reverse transcribing the mRNA population of a given cell or cell population, a cDNA library may be established.

"cDNA library", as used herein, refers to a collection of cDNAs representing the mRNAs expressed in a cell or cell population.

The term "cell population" refers to a grouping of cells isolated from a tissue or from a cell culture or from an environmental or forensic sample.

The term "mRNA", as used herein, refers to messenger RNA which is a transcription product of a gene expression process.

In the described methods, the nucleic acid can be provided by any means known in the art. Typically it is preferred in form of a sample. Said sample may be any type of cell- or nucleic acid-containing sample and may be a biological sample, such as a cellular or tissue sample, a body fluid sample, such as a blood sample, or a feces sample. In other various embodiments, the sample may be a food sample, an environmental sample, a forensic sample or a mold culture. The samples may be used as collected or may be subjected to various processing steps, such as purification and/or enrichment, before being used in the methods described herein.

The nucleic acid molecules that are analyzed can be any type of RNA, such as mRNA but also any non-coding RNA, such as rRNA, tRNA, asRNA, hnRNA, miRNA, ribozymes, siRNA, snoRNA, or snRNA. In various other embodiments, the nucleic acid analyzed is DNA, preferably genomic DNA. In preferred embodiments, the nucleic acid is genomic DNA or mRNA.

The nucleic acid may generally be of prokaryotic or eukaryotic origin. However, nucleic acids, such as genomic DNA or mRNA of eukaryotic origin are preferred.

While reference is generally made to "nucleic acid" herein, it should be understood that this term includes single nucleic acid molecules as well as mixtures of different nucleic acid molecules. For example, it can be genomic DNA or a pool of RNA molecules, in particular mRNA molecules, isolated from a cellular or tissue sample or any other sample type comprising cells, genomic DNA or RNA, in particular mRNA molecules, including body fluids.

In preferred embodiments of the invention, but without limitation, the nucleic acid molecules are eukaryotic mRNAs. Providing the mRNA may comprise isolating the mRNA from a eukaryotic cell sample. It is preferred that the mRNA comprises a multitude of different mRNA molecules, for example the mRNA population of a given cell, cell type or cell line. Methods for the isolation of RNA molecules from such samples are known in the art and kits for such mRNA isolation are commercially available.

In some embodiments, mRNA can be released from the cells by lysing the cells. Lysis can be achieved by, for example, heating the cells, or by the use of detergents, or a proteinase or other chemical methods, or by a combination of these. However, any suitable lysis method known in the art can be used. A mild lysis procedure can advantageously be used to prevent the release of nuclear chromatin, thereby avoiding genomic contamination of the cDNA library, and to minimize degradation of mRNA. For example, heating the cells at 72° C. for 2 minutes in the presence of Tween-20 is sufficient to lyse the cells while resulting in no detectable genomic contamination from nuclear chromatin. Alternatively, cells can be heated to 65 °C for 10 minutes in water (Esumi et al., Neurosci Res 60(4):439-51 (2008)); or 70° C for 90 seconds in PCR buffer II (Applied Biosystems) supplemented with 0.5% NP-40 (Kurimoto et al., Nucleic Acids Res 34(5):e42 (2006)). Lysis can also be achieved with a protease such as Proteinase K or by the use of chaotropic salts such as guanidine isothiocyanate (See, e.g., U.S. Publication No. 2007/0281313).

Eukaryotic mRNA derived from cells typically comprises a plurality of different mRNA molecules and in some embodiments comprises the complete cellular mRNA population. Accordingly, the steps of providing the eukaryotic mRNA and reverse transcribing the mRNA may be performed for a plurality of different mRNAs or mRNA populations, wherein for each mRNA/mRNA population a universal RT primer with a different unique identifier sequence is used, wherein the thus generated plurality of cDNA molecules is then combined for all subsequent method steps. By using primers with different unique identifier sequences for the reverse transcription procedure, also mRNA populations derived from different sources may be combined in all subsequent steps, while still being identifiable and distinguishable.

In the step of single stranded DNA synthesis a DNA polymerase is used. In case the analyzed nucleic acid is RNA a RNA-dependent DNA polymerase or a DNA polymerase that can use DNA as well as RNA as a template may be used. For reverse transcribing mRNA to form single-stranded cDNA, preferably reverse transcriptase (RT) is used.

Synthesis of cDNA from mRNA in the methods described herein can be performed directly on cell lysates or other sample types. In such embodiments, the reaction mix for reverse transcription is added directly to the sample. Alternatively and more preferably, the mRNA can be isolated from the sample and/or purified. This can help to reduce mitochondrial and ribosomal contamination. mRNA purification can be achieved by any method known in the art, for example, by binding the mRNA to a solid phase. Commonly used purification methods include paramagnetic beads (e.g. Dynabeads). Alternatively, specific contaminants, such as ribosomal RNA can be selectively removed using affinity purification.

As used herein, "dNTP" is understood as deoxynucleotide triphosphate which includes the natural or "standard" dNTPs, dATP, dCTP, dGTP, and dTTP. As used herein, dNTP also include natural and non-natural nucleotide analogs, such as fluorescently or otherwise chemically labeled nucleotides.

The term "ddNTP", as used herein, refers to dideoxynucleotides, also known as 2',3' dideoxynucleotides. The standard ddNTPS are abbreviated as ddGTP, ddATP, ddTTP and ddCTP. In ddNTPs the 3'-hydroxyl group is absent meaning that, after being added by a DNA polymerase to a growing nucleotide chain, no further nucleotides can be added as no phosphodiester bond can be created based on the fact that deoxyribonucleoside triphosphates (which are the building blocks of DNA) allow DNA chain synthesis to occur through a condensation reaction between the 5' phosphate (following the cleavage of pyrophospate) of the current nucleotide with the 3' hydroxyl group of the previous nucleotide. ddNTPs include natural and non-natural nucleotide analogs, such as fluorescently or otherwise chemically labeled nucleotides. In preferred embodiments, the ddNPTs are selected from the group consisting of ddATP, ddCTP, ddGTP, ddTTP or any combination thereof. In more preferred embodiments, the ddNTP is ddVTP, meaning a mixture of ddATP, ddCTP, and ddGTP, even more preferred an equimolar mixture of ddATP, ddCTP, and ddGTP.

As used herein, "3' end" means at any location on the oligonucleotide from and including the 3' terminus to the center of the oligonucleotide, usually at any location from and including the 3' terminus to about 10 bp from the 3' terminus, and more usually at any location from and including the 3' terminus to about 5 bp from the 3' terminus. More preferably, the term "3' end" refers to the last nucleotide at the opposite end of the 5' end.

As used herein, "5' end" means at any location on the oligonucleotide from and including the 5' terminus to the center of the oligonucleotide, usually at any location from and including the 5' terminus to about 10 bp from the 5' terminus, and more usually at any location from and including the 5' terminus to about 5 bp from the 5' terminus. The 5' end designates the end of the DNA or RNA strand that has the fifth carbon in the sugar-ring of the deoxyribose or ribose at its terminus. One or more phosphate groups may be attached to the 5' end.

Step f)ii), namely contacting the DNA with a linker molecule comprising a nucleic acid portion and a DNA ligase to ligate the DNA to the linker molecule, refers to the ligation of the DNA to a further molecule comprising a nucleic acid portion. The attachment of the DNA to the molecule is catalyzed by DNA ligase. Said ligase is well-known in the art. Preferably, the DNA is (directly) ligated to nucleic acid portion. More preferably, the 3' end of the DNA is ligated to the 5' end of the nucleic acid portion of the linker molecule. In various embodiments, the linker molecule is a DNA molecule.

The term "conditions that allow the generation of DNA", as used herein, refer to one or more parameters, such as temperature, salt concentration, pH etc., that influence the synthesis of DNA. These conditions also include suboptimal conditions, such that the amount of the generated DNA is not more than 90%, not more than 80%, not more than 70%, not more than 60% or not more than 50% of the amount generated under optimal conditions. In alternative embodiments, the suboptimal conditions comprise conditions under which the mutation rate after synthesis is increased by not more than 300%, not more than 100%, not more than 50% or not more than 10% compared to the mutation rate under optimal conditions. The optimal conditions may vary dependent on the polymerase which is used for DNA generation and are well-known in the art.

"Transcription" or "transcribed", as interchangeably used herein, refers to the process whereby information contained in a nucleotide sequence of DNA or RNA is transferred to a complementary single-stranded DNA sequence. Preferably, this process is achieved by using a DNA polymerase, such as reverse transcriptase.

In various embodiments, the reverse transcriptase (RT) is selected from the group consisting of MMLV RT, Avian Myeloblastosis Virus (AMV) RT, Rous Sarcoma Virus (RSV) RT, Human Immunodeficiency Virus (HIV) RT, Equine Infectous Anemia Virus (EIAV) RT, Rous associated Virus-2 (RAV2) RT, Avian Sarcoma Leukosis Virus (ASLV) RT, Maxima RT (Thermo Scientific), and RNaseH (-) RT, such as Maxima H minus RT (Thermo Scientific), SuperScript® RT, SuperScript® II RT, SuperScript® III RT and ThermoScript™ RT (Life Technologies). The RT may be provided and used in a buffer suitable for the reverse transcription reaction.

In various alternative embodiments, a DNA polymerase with reverse transcriptase activity, like Thermus thermophilus HB-8 (Tth) DNA polymerase, is used.

For transcribing the nucleic acid of interest into a complementary single-stranded DNA molecule, primers are used. To allow this, the primers used comprise a target-complementary region. This target-complementary region facilitates hybridization to the nucleic acid sequence under the synthesis conditions and allows elongation of the primer by the DNA polymerase. The target-complementary regions of the utilized primers typically are between 5 and 50 nucleotides in length. The length and sequence of said target-complementary region is selected such that the desired hybridization specificity under the given synthesis conditions is achieved.

The term "hybridization", as used herein, refers to the annealing process of a primer to a complementary sequence of a nucleic acid molecule.

In various preferred embodiments, the target-complementary region is located at the 3'-end of the primer to allow elongation. The 5'-end of such primers may comprise any one or more of a variety of target non-complementary regions that provide for different functionalities and include, without limitation, a unique identifier (barcode) sequence; a sequencing primer binding site and optionally one or more sequencing adapter sites; a 5' mono-phosphorylation or 5'-adenylation; a detectable label, preferably a fluorescent label; and a non-base spacer.

In various preferred embodiments, the primer comprises at its 3'-end the target-complementary region and a sequencing primer binding site 5' to the target-complementary region. Depending on the sequencing method employed, the primer may comprise one or more, typically two, sequencing adapter sites. These are in various embodiments located between the sequencing primer binding site and the target-complementary region. The location of the sequencing primer binding site 5' to the target complementary region ensures that after circularization, in the following sequencing step the newly synthesized (typically reverse transcribed) sequence is sequenced first. Further, the location of two sequencing adapter sites between the target complementary region and the sequencing primer binding site ensures that the to-be-sequenced part of the elongated and circularized primer is located between the two adapter sites. This is to be considered when designing the primer and deciding on orientation of the adapter sites.

The sequencing primer binding site is a sequence that is complementary to the sequence of the primer used for sequencing and designed such that it allows hybridization under sequencing conditions.

The term "non-elongated", as used herein in respect to primer, refers to a primer population which is not hybridized to the nucleic acid molecule of interest and is not processed by the DNA polymerase to synthesize a complementary nucleotide molecule of the nucleic acid of interest. Thus, the non-elongated primer population remains non-processed and maintains its chemical structure throughout the transcription step.

"Processed nucleotide", as used herein, refers to nucleotide molecules that comprise an amended chemical structure, such as the deletion of nucleotides, after a processing step, such as the exposure to an enzyme.

The term "sequencing adapter", as used herein, refers to a nucleic acid sequence that can specifically bind to oligo sequences or complementary adapters that are specific for the used sequencing system and, for example, facilitate capture onto a solid support or particle. The sequencing adapters may be sequencing technology-specific, i.e. if the Illumina sequencing protocol is to be used for the sequencing step, the sequencing adapters are Illumina adapters.

The terms "elongated primer", "extended primer" or "(synthesized) DNA molecule", as interchangeably used herein, refer to the primer after the DNA synthesis (transcription; elongation) and optionally also after site-specific cleavage. The term "non-extended primer", as used herein, refers to a primer that either does not hybridize to the target or is not elongated after hybridizing to a template nucleic acid molecule and thus does not contain any useful sequence information.

To allow identification, it is further preferred that the primer comprises a unique identifier sequence (barcode sequence). This is typically located adjacent to the sequencing primer binding-site. This sequence can be any sequence that is suitable and sufficient to allow identification. Suitable sequences are known in the art. In any case, the identifier sequence is selected such that it does not interfere with any following enzymatic treatment, hybridization and/or sequencing step/event. In certain embodiments, the barcode comprises a sequence of about 4 to about 50 nucleotides, of about 4 to about 25 nucleotides, of about 4 to about 20 nucleotides, of about 4 to about 15 nucleotides, of about 8 to about 12 nucleotides, or about 3, 4, 5, 6, 7, 8, 9, 10, 11, 12, 13, 14, 15, 16, 17, 18, 19, or 20 nucleotides. In certain embodiments, the barcode sequences used are generated by a pseudo-random sequence generator.

The primer may further comprise a mono-phosphorylation or an adenylation at its 5' end. The 5'-mono-phosphorylation or adenylation facilitates the circularization of the elongated primer by ssDNA ligase, as in the step of DNA circularization the 5' end of the elongated primer is ligated to the 3' end of the transcribed DNA sequence.

Alternatively, the primer may be enzymatically phosphorylated after elongation and before the circularization. Phosphorylation can, for example, be achieved by use of a polynucleotide kinase.

In various embodiments, the primer can further comprise a detectable label, preferably a fluorescent label. However, the label is not limited to fluorescent labels but can also be any other nucleic acid-compatible label known in the art. The label may facilitate detection, purification, or quantification of the primer or the generated cDNA molecules.

In still further embodiments, the primer may comprise a non-base spacer, which can be any non-nucleotide spacer known in the art, e.g. a carbon spacer that functions as an effective blocking agent against polymerase extension. Exemplary spacers can be, without limitation, hexa-ethyleneglycol spacers, tri-ethyleneglycol spacers, C3 spacers, 1',2'-dideoxyribose spacers, or photo-cleavable spacers. The function of the spacer is to hinder unwanted circular amplification of the cDNA in the later sequencing process, e.g. during cluster generation on an Illumina flowcell.

In preferred embodiments of the invention, the universal RT primer comprises in 3' to 5' orientation the target-complementary region, a sequencing adapter, an optional non-base blocking spacer, an optional label, an optional second sequencing adapter, a sequencing primer binding-site, and an identifier barcode sequence. In other embodiments of the invention, the universal RT primer comprises in 3' to 5' orientation a target-complementary sequence, an identifier barcode sequence, a sequencing primer binding-site, a sequencing adapter, an optional non-base blocking spacer, an optional label, and an optional second sequencing adapter.

The concrete primer sequence will depend on the target nucleic acid. However, it may in various embodiments be preferable to use a universal primer that hybridizes to a multitude of different target molecules which are then later identified by sequencing. "Universal primer", as used herein, thus relates to a primer that is not specific for a single target sequence, but rather indiscriminately binds to two or more different target sequences, typically a large population of different targets. This is usually achieved by either randomizing the universal primer target-complementary region sequence or by using a target-complementary sequence that recognizes a sequence element present in a multitude of different target molecules, such as a common sequence motif.

A randomized sequence may be a poly(dN) or (dN)₆₋₁₀ sequence, wherein N is A, G, T or C.

A universal target-complementary region that hybridizes to all the poly-A tail present in all eukaryotic mRNA molecules is a poly(dT) sequence.

Consequently, in preferred embodiments where the nucleic acid of interest is a eukaryotic mRNA, the primer is a universal RT primer that under condition of a reverse transcriptase enzymatic activity acts as a starting point for template-directed cDNA synthesis. Such a primer may comprise at its 3'-end a target-complementary region comprising or consisting of a poly(dT)-dV-3'OH or poly(dT)-dV-dN-3'OH sequence to facilitate hybridization to the polyadenylation sequence of the mRNA, wherein the poly(dT) sequence is 5 to 50 nucleotides in length and V is A, C or G and N is A, C, G or T. The V or VN sequence at its 3'-end is a degenerate sequence, i.e. the RT primer includes a population of primers that differ in this position in that they have either an A, C or G nucleotide for V and, if present, any one of A, G, C or T for N. This allows recognition of the end of the poly-A tail of the mRNA molecule and recognition of the 3'-end of the gene-specific mRNA region, which in turn ensures that reverse transcription of the gene-specific mRNA sequence is correctly initiated. As already described above, the poly(dT) sequence should be long enough to allow specific hybridization to the poly-A tail of the mRNA molecules. Typically, the length of the poly(dT) sequence is thus 10 to 30, preferably 15 to 25 T nucleotides.

The dNTP mixture used for the reverse transcription step comprises dATP, dGTP, dCTP, and dTTP. Generally, the amounts of dATP, dGTP, dCTP and dTTP are essentially equimolar. "Essentially equimolar", as used in this context, means that the respective nucleotides or nucleotide combinations are used in nearly identical amounts. In various embodiments, "essentially equimolar" means that the variance in the concentrations of the nucleotides in the mixture is no more than about ± 5%.

The term "dATP, dCTP, dGTP, or dTTP nucleotide comprising a modified 3'-moiety", as used herein, refers to dNTPs those carbon atom at the position 3 of the desoxyribose is not hydroxylated. The structure of the modified dNTP may be as shown in formula (I): 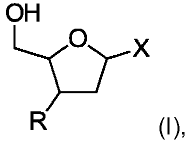
wherein X is adenine, guanine, cytosine, thymine or derivatives thereof; and
R is nothing, a linear or branched, substituted or unsubstituted alkyl, linear or branched, substituted or unsubstituted heteroalkyl, linear or branched, substituted or unsubstituted alkenyl, linear or branched, substituted or unsubstituted heteroalkenyl, linear or branched, substituted or unsubstituted alkynyl, linear or branched, substituted or unsubstituted heteroalkynyl, substituted or unsubstituted cycloalkyl, substituted or unsubstituted heterocycloalkyl, substituted or unsubstituted aryl, or substituted or unsubstituted heteroaryl, linear or branched, substituted or unsubstituted alkylaryl, linear or branched, substituted or unsubstituted heteroalkylaryl, each having up to 20 carbon atoms, or phosphoryl.

The term "alkyl," by itself or as part of another substituent, means, unless otherwise stated, a straight (i.e. unbranched) or branched chain, or combination thereof, which is be fully saturated and can include di- and multivalent radicals, having the number of carbon atoms designated (i.e. C₁-C₁₀ means one to ten carbons). Examples of saturated hydrocarbon radicals include, but are not limited to, groups such as methyl, ethyl, n-propyl, isopropyl, n-butyl, t-butyl, isobutyl, sec-butyl, cyclohexyl, (cyclohexyl)methyl, cyclopropylmethyl, homologs and isomers of, for example, n-pentyl, n-hexyl, n-heptyl, n-octyl, and the like.

The term "alkylene" by itself or as part of another substituent means a divalent radical derived from an alkyl, as exemplified, but not limited, by -CH₂CH₂CH₂CH₂-. Typically, an alkyl (or alkylene) group will have from 1 to 24 carbon atoms, with those groups having 10 or fewer carbon atoms being preferred in the present invention. A "lower alkyl" or "lower alkylene" is a shorter chain alkyl or alkylene group, generally having eight or fewer carbon atoms.

The term "heteroalkyl," by itself or in combination with another term, means, unless otherwise stated, a stable straight or branched chain, or cyclic hydrocarbon radical, or combinations thereof, consisting of at least one carbon atoms and at least one heteroatom selected from the group consisting of O, N, P, Si and S, and wherein the nitrogen and sulfur atoms may optionally be oxidized and the nitrogen heteroatom may optionally be quaternized. The heteroatom(s) O, N, P and S and Si may be placed at any interior position of the heteroalkyl group or at the position at which the alkyl group is attached to the remainder of the molecule. Examples include, but are not limited to, -CH₂-CH₂-O-CH₃, -CH₂-CH₂-NH-CH₃, -CH₂-CH₂-N(CH₃)-CH₃, - CH₂-S-CH₂-CH₃, -CH₂-CH₂, -S(O)-CH₃, -CH₂-CH₂-S(O)₂-CH₃, - CH=CH-O-CH₃, -Si(CH₃)₃, -CH₂-CH=N-OCH₃, -CH=CH-N(CH₃)-CH₃, O-CH₃, -O-CH₂-CH₃, and -CN. Up to two heteroatoms may be consecutive, such as, for example, -CH₂-NH-OCH₃ and -CH₂-O-Si(CH₃)₃. Similarly, the term "heteroalkylene" by itself or as part of another substituent means a divalent radical derived from heteroalkyl, as exemplified, but not limited by, -CH₂-CH₂-S-CH₂-CH₂- and -CH₂-S-CH₂-CH₂-NH-CH₂-. For heteroalkylene groups, heteroatoms can also occupy either or both of the chain termini (e.g., alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula -C(O)₂R'- represents both -C(O)₂R'- and -R'C(O)₂-. As described above, heteroalkyl groups, as used herein, include those groups that are attached to the remainder of the molecule through a heteroatom, such as -C(O)R', -C(O)NR', - NR'R", -OR', -SR', and/or -SO₂R'. Where "heteroalkyl" is recited, followed by recitations of specific heteroalkyl groups, such as -NR'R" or the like, it will be understood that the terms heteroalkyl and -NR'R" are not redundant or mutually exclusive. Rather, the specific heteroalkyl groups are recited to add clarity. Thus, the term "heteroalkyl" should not be interpreted herein as excluding specific heteroalkyl groups, such as -NR'R" or the like.

The terms "cycloalkyl" and "heterocycloalkyl", by themselves or in combination with other terms, represent, unless otherwise stated, cyclic versions of "alkyl" and "heteroalkyl", respectively. Additionally, for heterocycloalkyl, a heteroatom can occupy the position at which the heterocycle is attached to the remainder of the molecule. Examples of cycloalkyl include, but are not limited to, cyclopentyl, cyclohexyl, 1-cyclohexenyl, 3-cyclohexenyl, cycloheptyl, and the like. Examples of heterocycloalkyl include, but are not limited to, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like. A "cycloalkylene" and "heterocycloalkylene" refer to a divalent radical derived from cycloalkyl and heterocycloalkyl, respectively.

The term "aryl" means, unless otherwise stated, a polyunsaturated, aromatic, hydrocarbon substituent which can be a single ring or multiple rings (preferably from 1 to 3 rings) which are fused together or linked covalently. The term "heteroaryl" refers to aryl groups (or rings) that contain from one to four heteroatoms selected from N, O, and S, wherein the nitrogen and sulfur atoms are optionally oxidized, and the nitrogen atom(s) are optionally quaternized. A heteroaryl group can be attached to the remainder of the molecule through a carbon or heteroatom. Non-limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. Substituents for each of the above noted aryl and heteroaryl ring systems are selected from the group of acceptable substituents described below. "Arylene" and "heteroarylene" refers to a divalent radical derived from a aryl and heteroaryl, respectively.

The term "arylalkyl" is meant to include those radicals in which an aryl group is attached to an alkyl group (e.g., benzyl, phenethyl, pyridylmethyl and the like). The term "heteroarylalkyl" includes the above described groups wherein one or more carbon atoms of the alkyl or aryl portion (e.g., a methylene group) are replaced by, for example, an oxygen atom (e.g., phenoxymethyl, 2-pyridyloxymethyl, 3-(1-naphthyloxy)propyl, and the like).

Where a heteroalkyl, heterocycloalkyl, or heteroaryl includes a specific number of members (e.g. "3 to 7 membered"), the term "member" refers to a carbon or heteroatom.

Each of the above terms (e.g., "alkyl," "heteroalkyl," "aryl" and "heteroaryl") are meant to include both substituted and unsubstituted forms of the indicated radical. Preferred substituents for each type of radical are provided below.

Substituents for the alkyl, heteroalkyl, alkylene, alkenyl, heteroalkylene, heteroalkenyl, alkynyl, cycloalkyl, heterocycloalkyl, cycloalkenyl, and heterocycloalkenyl radicals can be one or more of a variety of groups selected from, but not limited to: -OR', =O, =NR', =N-OR', -NR'R", -SR', -halogen, -SiR'R"R"', -OC(O)R', -C(O)R', - CO₂R', -CONR'R", -OC(O)NR'R", -NR"C(O)R', -NR'-C(O)NR"R''', -NR"C(O)₂R', -NR-C(NR'R"R"')=NR"", -NR-C(NR'R")=NR"', -S(O)R', -S(O)₂R', - S(O)₂NR'R", -NRSO₂R', -CN and -NO₂ in a number ranging from zero to (2 m'+1), where m' is the total number of carbon atoms in such radical. R', R", R"' and R"" each preferably independently refer to hydrogen, substituted or unsubstituted heteroalkyl, substituted or unsubstituted cycloalkyl, substituted or unsubstituted heterocycloalkyl, substituted or unsubstituted aryl (e.g., aryl substituted with 1-3 halogens), substituted or unsubstituted alkyl, alkoxy or thioalkoxy groups, or arylalkyl groups. When a compound of the invention includes more than one R group, for example, each of the R groups is independently selected as are each R', R", R'" and R"" groups when more than one of these groups is present. When R' and R" are attached to the same nitrogen atom, they can be combined with the nitrogen atom to form a 4-, 5-, 6-, or 7-membered ring. For example, -NR'R" is meant to include, but not be limited to, 1-pyrrolidinyl and 4-morpholinyl. From the above discussion of substituents, one of skill in the art will understand that the term "alkyl" is meant to include groups including carbon atoms bound to groups other than hydrogen groups, such as haloalkyl (e.g., -CF₃ and - CH₂CF₃) and acyl (e.g., -C(O)CH₃, -C(O)CF₃, -C(O)CH₂OCH₃, and the like).

Similar to the substituents described for the alkyl radical, substituents for the aryl and heteroaryl groups are varied and are selected from, for example: halogen, -OR', - NR'R", -SR', -halogen, -SiR'R"R"', -OC(O)R', -C(O)R', -CO₂R', -CONR'R", - OC(O)NR'R", -NR"C(O)R', -NR'-C(O)NR"R"', -NR"C(O)₂R', -NR-C(NR'R"R"')=NR"", -NR-C(NR'R")=NR"', -S(O)R', -S(O)₂R', -S(O)₂NR'R", - NRSO₂R', -CN and -NO₂, -R', -N₃, -CH(Ph)₂, fluoro(C₁-C₄)alkoxy, and fluoro(C₁-C₄)alkyl, in a number ranging from zero to the total number of open valences on the aromatic ring system; and where R', R", R"' and R"" are preferably independently selected from hydrogen, substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl. When a compound of the invention includes more than one R group, for example, each of the R groups is independently selected as are each R', R", R'" and R"" groups when more than one of these groups is present.

As used herein, the term "heteroatom" or "ring heteroatom" is meant to include oxygen (O), nitrogen (N), sulfur (S), phosphorus (P), and silicon (Si).

The term "alkenyl" as used herein is a hydrocarbon group of from 2 to 20 carbon atoms with a structural formula containing at least one carbon-carbon double bond. Asymmetric structures such as (A1A2)C=C(A3A4) are intended to include both the E and Z isomers. The alkenyl group can be substituted with one or more groups including, but not limited to, alkyl, cycloalkyl, alkoxy, alkenyl, cycloalkenyl, alkynyl, aryl, heteroaryl, aldehyde, amino, carboxylic acid, ester, ether, halide, hydroxy, ketone, azide, nitro, silyl, sulfo-oxo, or thiol, as described herein.

The term "cycloalkenyl" as used herein is a non-aromatic carbon-based ring composed of at least three carbon atoms and containing at least one carbon-carbon double bound, i.e., C=C. Examples of cycloalkenyl groups include, but are not limited to, cyclopropenyl, cyclobutenyl, cyclopentenyl, cyclopentadienyl, cyclohexenyl, cyclohexadienyl, norbornenyl, and the like. The cycloalkenyl group can be substituted or unsubstituted. The cycloalkenyl group can be substituted with one or more groups including, but not limited to, alkyl, cycloalkyl, alkoxy, alkenyl, cycloalkenyl, alkynyl, aryl, heteroaryl, aldehyde, amino, carboxylic acid, ester, ether, halide, hydroxy, ketone, azide, nitro, silyl, sulfo-oxo, or thiol as described herein.

The term "alkynyl" as used herein is a hydrocarbon group of 2 to 20 carbon atoms with a structural formula containing at least one carbon-carbon triple bond. The alkynyl group can be unsubstituted or substituted with one or more groups including, but not limited to, alkyl, cycloalkyl, alkoxy, alkenyl, cycloalkenyl, alkynyl, aryl, heteroaryl, aldehyde, amino, carboxylic acid, ester, ether, halide, hydroxy, ketone, azide, nitro, silyl, sulfo-oxo, or thiol, as described herein.

In preferred embodiments, the step c) of degrading the non-elongated primer includes an enzymatic digestion, preferably digestion by an exonuclease, more preferably by an exonuclease of the DnaQ-like (or DEDD) 3'-5' exonuclease superfamily, and even more preferably by Exonuclease T (Exo T). This enzyme is also described as the 3' to 5' exonuclease not catalyzing the digestion of a nucleic acid having at its 3' terminal end a ddATP, ddCTP, ddGTP, ddTTP, or a nucleotide of dATP, dCTP, dGTP, or dTTP comprising a modified 3'-moiety. Exonuclease T (Exo T), also known as RNase T, is a single-stranded RNA or DNA specific nuclease that requires a free 3' terminus and removes nucleotides in the 3'→ 5' direction. In other preferred embodiments, the exonuclease degrading the non-elongated primer is Exonuclease I (Exo I). Exonuclease I breaks apart single-stranded DNA in a 3' → 5' direction, releasing deoxyribonucleoside 5'-monophosphates one after another. It does not cleave DNA strands without terminal 3'-OH groups. Exonuclease I hydrolyzes single-stranded DNA in the 3'→5' direction, releasing 5'-mononucleotides and leaving the terminal 5'-dinucleotide intact. Hydrolysis is processive and cannot proceed if the 3' terminus is modified. In more preferred embodiments, Exonuclease T is used for digestion of primers in a method wherein the DNA comprises ddNTPs and Exonuclease I is used for digestion of primers in a method wherein the DNA comprises dNTPs with a modified 3' moiety. In various embodiments, the exonuclease is T4 DNA polymerase or a Klenow fragment.

In preferred embodiments, the 3' to 5' exonuclease which removes the ddATP, ddCTP, ddGTP or ddTTP nucleotide or the dATP, dCTP, dGTP, or dTTP nucleotide comprising the modified 3'-moiety from the DNA is a non-processive enzyme. This enzyme is also described as the 3' to 5' exonuclease catalyzing the digestion of a nucleic acid having at its 3' terminal end a ddATP, ddCTP, ddGTP, ddTTP, or a nucleotide of dATP, dCTP, dGTP, or dTTP comprising a modified 3'-moiety. The term "processivity", as used herein, refers to an enzyme that is able to catalyze consecutive reactions without releasing its substrate. The removing step may be achieved by the proofreading activity of DNA polymerases. A 3'→ 5' proofreading exonuclease domain is intrinsic to most DNA polymerases. It allows the enzyme to check each nucleotide during DNA synthesis and excise mismatched nucleotides in the 3' to 5' direction. The proofreading domain also enables a polymerase to remove unpaired 3' overhanging nucleotides to create blunt ends. In other preferred embodiments, the 3' to 5' exonuclease is thermostable. This means that the exonuclease possesses enzymatic activity (for example, at least 30%, 40% or 50% of its activity shown at room temperature) at more than 40°C, more than 50°C, more than 60°C, more than 80°C or more than 90°C. In more preferred embodiments, the 3' to 5' exonuclease is the Phusion polymerase. In various embodiments, the exonuclease is a polymerase having proofreading activity, such as bacterial DNA polymerases I, II or III or the eukaryotic DNA polymerases delta or epsilon. In other various embodiments, the exonuclease is Exonuclease I.

As already described above, only the provision of the nucleic acid molecule, i.e. typically its isolation, and DNA synthesis have to be performed for individual samples, with these steps being easily automatable. All subsequent steps can then be performed on a pool of elongated primer molecules from different samples. In order to facilitate pooling of a multitude of samples, the universal primer used for each sample may comprise a unique identifier (barcode) sequence. This sequence allows identification of each DNA population generated by use of a given primer and thus serves as an identification means for the origin of the DNA, i.e. the sample it is derived from, even in case the DNA from multiple samples has been combined for sequencing analysis.

In embodiments where the nucleic acid is (eukaryotic) mRNA, the mRNA may digested once the cDNA has been synthesized, i.e. prior to step c) and/or d) and/or e). The digestion step may be carried out by contacting the mRNA molecules with a ribonuclease (commonly abbreviated RNase), which catalyzes the degradation of the RNA molecules into smaller fragments, in particular single nucleotides. As the RNase is RNA-specific, the cDNA molecules are unaffected by this treatment and stay intact. This way, the mRNA molecules do not interfere with the following sequencing reaction. In various embodiments of the invention, the RNAse may be selected from the group consisting of RNAse A, RNAse H, RNAse I, RNAse T and RNAse activity of any Reverse Transcriptase. Alternatively, mRNA can be degraded by addition of NaOH and heating or by heating in a buffer alone.

The following optional step of purifying the DNA molecules from RNA molecules, enzymes, unused primers or any degradation products of the aforementioned components can be performed by any nucleic acid purification method known in the art. Suitable techniques involve, without limitation, precipitation, centrifugation, chromatography (affinity or size exclusion), silica column purification, electrophoretic techniques, such as gel electrophoresis, and the like. In various embodiments of the invention, the DNA purification comprises precipitation, chromatography, and/or gel electrophoresis.

The purified DNA molecules are then circularized by contacting the DNA with a single-stranded DNA ligase (ssDNA ligase), which fuses the 3'OH end to the mono-phosphorylated or adenylated 5' end of the DNA molecule. The single-stranded DNA ligase (ssDNA ligase) may be selected from the group consisting of CircLigase ® (Epicentre), CircLigase II ® (Epicentre), Mth thermostable ligase (NEB) and T4 RNA ligase I/II ® (New England Biolabs), preferably is CircLigase ® (Epicentre).

In some embodiments, gel electrophoresis is used to separate circularized DNA from linear DNA or to quantify circularized DNA, as known from prior art, wherein the gel can be made of a variety of gel matrix materials, including polyacrylamide, agarose, polyacrylamide-agarose composites, and the like.

In other embodiments, the circularized DNA molecules are purified by using commonly known precipitation methods, such as ethanol precipitation, precipitation by isopropanol, polyethylene glycol in aqueous sodium chloride or spin column-based solid phase extraction.

In various embodiments, the circularized DNA molecules are purified by chromatography, which refers to a process in which a chemical mixture carried by a liquid or gas is separated into components as a result of differential distribution of the chemical entities as they flow around or over a stationary liquid or solid phase.

In various embodiments, the ratio between the ddNTPs or the dNTP nucleotides comprising a modified 3'-moiety and the respective dNTPs is in the range of 1:1 to 1:1000, preferably 1:5 to 1:20.

In various embodiments, the ddNTP nucleotides consist of ddATP, ddCTP, ddGTP and any combination thereof, preferably ddVTPs or the dNTP nucleotides comprising a modified 3'-moiety consist of modified dATP, dCTP, dGTP and any combination thereof, preferably modified dVTPs. In preferred embodiments, the ddNTP nucleotides or the dNTPs nucleotides comprising a modified 3'-moiety do not comprise ddTTP or modified dTTP. Thus, if only ddATP, ddCTP, ddGTP and any combination thereof or modified dATP, dCTP, dGTP and any combination thereof are used to block primer elongation, the elongation will not stop in the poly-A region of the nucleic acid to be investigated but will stop in the protein coding region.

In various embodiments, steps e) and f)i) or steps e) and f)ii) are accomplished at the same time. This means that the elongated and blocked primer DNA is contacted with the 3' to 5' exonuclease and the ssDNA ligase to circularize the DNA; or with a linker molecule comprising a nucleic acid portion and a DNA ligase to ligate the DNA to the linker molecule in parallel. Preferably, the 3' to 5' exonuclease is a non-processive exonuclease meaning that the enzyme is not able to catalyze consecutive reactions without releasing its substrate. The processivity of the exonuclease may be determined by its specific activity. Methods to processivity are well-known in the art.

In various embodiments, the invention further comprises the step of quantifying the circularized DNA prior to the sequencing step. The quantifying may comprise quantitative PCR (qPCR) or digital PCR (dPCR), optionally using primers capable of only amplifying circular DNA and/or a linear control template for absolute quantification.

In various embodiments the method further comprises the step of degrading linear DNA by contacting the DNA with an exonuclease after the circularization step but prior to the sequencing step and optionally the quantifying step. In various embodiments, the exonuclease may be selected from the group consisting of phosphodiesterase, nuclease, exonuclease I, exonuclease VII, an exonuclease activity of *E. coli* DNA polymerase I, an exonuclease activity of a Klenow fragment of DNA polymerase I, an exonuclease activity of T4 DNA polymerase ® (NEB), an exonuclease activity of T7 DNA polymerase ® (NEB), an exonuclease activity of Taq DNA polymerase ® (Roche, NEB, Qiagen or Life Technologies), an exonuclease activity of Deep Vent ® DNA polymerase (NEB), and an exonuclease activity of Ventr ® DNA polymerase (NEB).

In various embodiments of the invention, a polymerase chain reaction (PCR) is performed before sequencing to increase the amount of material to be sequenced. The PCR primers used for said amplification may bind to the sequencing adapter sequences of the circular cDNA. This can be important if the initial amount of RNA is limited, e.g. because it was derived from a single cell.

In other various embodiments, the DNA is amplified using rolling circle amplification (RCA). Specific variants of RCA to avoid concatemeric products are preferred. Such methods are known in the art, for example from the disclosure of Gu and Breaker (Gu, H. and Breaker, R.R. (2013), BioTechniques, vol. 54, no. 6, pages 337-343). Alternatively, in order to avoid concatemeric products of RCA may be exposed to restriction enzyme based digestion.

In various embodiments of the invention, the sequencing is deep sequencing, preferably next generation deep sequencing.

The circularized DNA molecule is then sequenced. For sequencing, any sequencing reaction known in the art can be used. The term "sequencing", as used herein, generally means a process for determining the order of nucleotides in a nucleic acid. "Sequencing", as used herein, comprises de novo identification of unknown sequences as well as identification of known sequences or sequence changes, e.g. mutations. A variety of methods for sequencing nucleic acids is well known in the art. Such sequencing methods include the Sanger method of dideoxy-mediated chain termination as described, for example, in Sanger et al., Proc. Natl. Acad. Sci. 74:5463 (1977) or next generation sequencing or higher generation sequencing.

The term "next generation sequencing", as used herein, refers to sequencing technologies having increased throughput as compared to traditional Sanger- and capillary electrophoresis-based approaches, for example with the ability to generate hundreds of thousands or millions of relatively short sequence reads at a time. Some examples of next generation sequencing techniques include, but are not limited to, sequencing by synthesis, sequencing by ligation, and sequencing by hybridization. Examples of next generations sequencing methods include pyrosequencing as used by the GS Junior and GS FLX Systems (454 Life Sciences), sequencing by synthesis as used by Illumina's Miseq and Solexa system, the SOLiD™ (Sequencing by Oligonucleotide Ligation and Detection) system (Life Technologies Inc.), and Ion Torrent Sequencing systems such as the Personal Genome Machine or the Proton Sequencer (Life Technologies Inc), and nanopore sequencing systems (Oxford nanopore). Alternatively, the sequencing method may Single Molecule, Real-Time (SMRT) Sequencing as established by Pacific Biosciences.

The term "deep sequencing ", as used herein, refers to nucleic acid sequencing to a depth that allows each base to be read multiple times from independent nucleic acid molecules (e.g., a large number of template molecules is sequenced relative to the length of the sequence) and allows sequencing of thousands of molecules simultaneously, thereby allowing to characterize complex pools of nucleic acid molecules and increasing sequencing accuracy. Deep sequencing of the transcriptome, also known as RNA-Seq, provides both the sequence and frequency of contained RNA molecule species that are present at any particular time in a specific cell type, tissue or organ.

In various embodiments, steps of the method of the invention are carried out in one container or vial. This may include that steps a), b) c), e), and f)i) or f)ii) are subsequently executed in the same container. If steps g) and h) are also implemented, they may be performed in the same container as steps a), b) c), e), and f)i) or f)ii).

In another aspect, the present invention is directed to a kit for gene analysis, preferably gene expression profiling, comprising
(A) DNA polymerase;
(B) one or more primers comprising a target-complementary region;
(C) a composition comprising dNTPs;
(D) a composition comprising ddATP, ddCTP, ddGTP, ddTTP or a dATP, dCTP, dGTP, or dTTP nucleotide comprising a modified 3'-moiety or any combination thereof, optionally in form of a mixture with the composition of (C);
(E) 3' to 5' exonuclease not catalyzing the digestion of a nucleic acid having at its 3' terminal end a ddATP, ddCTP, ddGTP, ddTTP, or a nucleotide of dATP, dCTP, dGTP, or dTTP comprising a modified 3'-moiety, preferably Exonuclease T and/or Exonuclease I;
(F) ssDNA ligase; and
(G) 3' to 5' exonuclease catalyzing the digestion of a nucleic acid having at its 3' terminal end a ddATP, ddCTP, ddGTP, ddTTP, or a nucleotide of dATP, dCTP, dGTP, or dTTP comprising a modified 3'-moiety.

The kits of the invention comprise in various embodiments the reagents that allow producing DNA libraries of circular DNA molecules that can directly be used in high-throughput next generation (deep) sequencing without prior amplification.

The DNA polymerase may be a RNA-dependent DNA polymerase, in particular a reverse transcriptase, such as those disclosed above in connection with the inventive methods.

The one or more primers may each be defined as described above in connection with the inventive methods. In various embodiments, the primers are universal RT primers comprising a target-complementary region at the 3' end comprising or consisting of a poly(dT)-dV-3'OH or a poly(dT)-dV-dN-3'OH sequence, wherein the poly(dT) sequence is 5 to 50 nucleotides in length and V is A,C or G and N is A, C, G or T.

The kit of the application may further comprise Exonuclease T and/or Exonuclease I.

In various embodiments, the kit may further comprise an exonuclease to degrade linear DNA.

In various embodiments, the kit may further comprise chemicals or means useful for DNA isolation/purification, all of which are well-known in the art.

In preferred embodiments, the kit may further comprise a ribonuclease for mRNA digestion.

In further embodiments, the kit may also comprise a linear DNA and control template primers capable of amplifying only circular DNA for qPCR quantification.

In still further embodiments, the kit may comprise sequencing primers. The term "sequencing primer", as used herein, refers to a single-stranded oligonucleotide generally with a free 3' -OH group capable of acting as a point of initiation for template-directed DNA synthesis within the sequencing reaction under suitable conditions for example, buffer and temperature, in the presence of a dNTP mixture and a polymerase. The length of the primer typically varies from 10 to 40, preferably 15 to 30 nucleotides. The primers are usually selected such that they hybridize to the respective binding site in the generated DNA molecules under sequencing conditions.

It is understood that all embodiments disclosed herein in relation to the inventive methods are, insofar possible, equally applicable to the kits of the invention and vice versa.

All documents cited are herein incorporated by reference in their entirety.

The invention is further illustrated by the following non-limiting examples.

### Examples

### Materials and Methods

### Oligos from IDT (smallest scale available, standard purification):

CircMrna 1' (barcode underlined) 
/5Phos/GATCAGATCGGAAGAGCGTCGTGTAGGGAAAGAGTGTAGATCTCGGTGG
TCGCCGTATCAAGCAGAAGACGGCATACGAGATTTTTTTTTTTTTTTTTT (SEQ ID
NO:1)
CircMrna 2' (barcode underlined) 
/5Phos/GACGTAGATCGGAAGAGCGTCGTGTAGGGAAAGAGTGTAGATCTCGGTG
GTCGCCGTATCAAGCAGAAGACGGCATACGAGATTTTTTTTTTTTTTTTTV (SEQ ID
NO:2)
SBS3 fwd (qPCR): AGATCGGAAGAGCGTCGTGTAGG (SEQ ID NO:3)
circ qPCR rev (qPCR): AATGATACGGCGACCACCGAGAT (SEQ ID NO:4)
CircMrna 8 (this primer was used as RT Primer for the MiSeq Run):

GGTGAAGATCGGAAGAGCGTCGTGTAGGGAAAGAGTGTAGATCTCGGTGGTCGC
CGTATCAAGCAGAAGACGGCATACGAGATTTTTTTTTTTTTTTTTTV (SEQ ID NO:5)

### 1. Purify total RNA from HEK293T cells

- trypsinize a confluent T175-flask of HEK293T cells with 2.5 ml Trypsin 0.05%;
- add 6.5 ml medium, resuspend, aliquot 500 µl per tube, pellet at 1000 g;
- resuspend pellets in 1 ml trizol per eppi, freeze all but 2 tubes at -20°C;
- follow the normal trizol protocol by the manufacturer, resuspend in 20 µl water per pellet; and
- measure concentration using nanodrop, which should be 1-2 µg/µl

### 2. AMV Reverse Transcription (8 reactions in a PCR stripe):

| | |
|---|---|
| 2 µl | RNA 1-2 µg/µl |
| 1 µl | PRIMER CircMrna 2' 0.1 µM |
| 0.5 µl | dNTP 10 mM |
| 1 µl | stop ddVTP mix 1 mM (0.33 mM ddATP, 0.33 mM ddCTP, 0.33 mM ddGTP in water) |
| 3.75 µl | water |
| -mix- | |

PCR cycler:

| |
|---|
| 5 min 65° |
| 1 min ice |

| | |
|---|---|
| 1 µl | 10x AMV buffer (NEB) |
| -mix- | |
| 0.25 µl | ribolock |
| 0.5 µl | AMV RT (NEB) |
| -mix 10x- | |

PCR cycler:

| |
|---|
| 60 min 42° |
| 5 min 85° |

### 3. ExoT digest (in same PCR stripe):

| | |
|---|---|
| 10 µl | RT reaction |
| 2 µl | NEB4 |
| 6 µl | water |
| -mix- | |
| 1 µl | ExoT (NEB) |
| - mix 10x- | |

PCR cycler:
1 h 25°

### 4. Column purification:

- add 500 µl binding buffer (Analytik Jena PCR purification kit) to a miniprep silica column;
- add the whole reaction, mix 4x with 1 ml pipette;
- spin for 2 min at 12000 g;
- put column in Eppendorf DNA-lobind 1.5 ml tube;
- add 17 µl water to the center of the membrane;
- incubate for 1 min at RT; and
- spin for 1min at 8000 g

### 5. Circularization:

Prepare 10x mastermix:

| | |
|---|---|
| 5 µl | ATP 1 mM (epicentre) |
| 5 µl | MnCl2 50 mM (epicentre) |
| 10 µl | 10x circligase buffer (epicentre) |

Fresh pre-diluted Phusion polymerase:

| | |
|---|---|
| 1 µl | 10x circligase buffer (epicentre) |
| 5.7 µl | water |
| -mix- | |
| 3.3 µl | Phusion polymerase (Thermo) |
| -mix 10x- | |

Prepare individual reactions in Eppendorf DNA-lobnd 1.5 ml tubes:

| | |
|---|---|
| 4 µl | mastermix |
| 14 µl | column eluate |
| 1 µl | Pre-diluted Phusion polymerase |
| 1 µl | CircLigase I (epicentre) |
| - mix 10x- | |

In eppendorf 1.5 ml thermoblock with heated lid:
12-16 h 60°
10 min 80° (pre-heat thermoblock while sample on ice)

### 6. Exol digestion for qPCR in Eppendorf DNA-Iobind 1.5ml tubes:

| | |
|---|---|
| 1 µl | NEBuffer4 |
| 1 µl | circularization reaction (keep the rest at 4°C until sequencing) |
| 7 µl | water |
| 1 µl | Exol (NEB) |

In eppendorf 1.5ml thermoblock with heated lid:
1 h 37°
20 min 80° (pre-heat thermoblock while sample on ice)

### 7. qPCR quantification

- prepare fresh standard dilutions in Eppendorf DNA-lobind 1.5ml tubes:
linear control oligo 1E-8 M, 1E-10 M , 1E-12 M, 1E-14 M (serial 1:100 dilutions 10µl + 990µl, initial 1 E-8 or 1 E-6 dilution should be in the RNA-seq box, don't reuse lower dilutions)
- prepare 30x mastermix:

| | |
|---|---|
| 150 µl | FastSYBR 2x master mix |
| 1.5 µl | Fwd primer 100 µM (SBS3 fwd) |
| 1.5 µl | Rev primer 100 µM (circ qPCR rev) |
| 87 µl | water |

in qPCR plate, in duplicates:

| | |
|---|---|
| 2 µl | Exo reaction / std dilution |
| 8 µl | mastermix |
| -mix- | |

- run normal program in qPCR cycler

### 8. Sequencing

- when starting a normal MiSeq run, add all circularization samples to the last dilution of other PCR libraries before pipetting the final mix into the MiSeq reagent cassette;
- the total volume of the final dilution has to be adjusted (use 20µl less Hyb1 buffer per RNA-seq sample in the final dilution); and
- no denaturing or pre-dilution is required

### Example 1: Use of different ddNTP concentrations

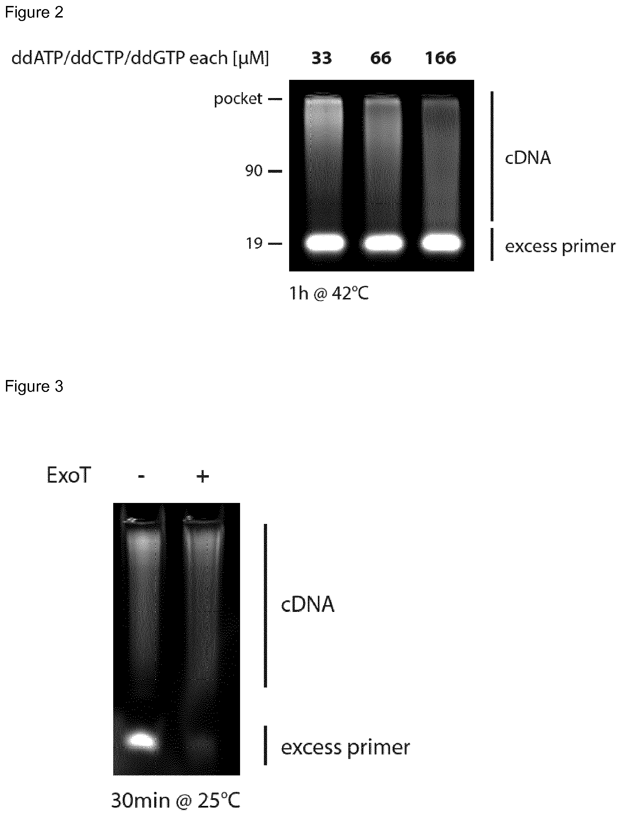

Sample RNAs are reverse transcribed using a polymerase (AMV RT), dNTPs and a mixture of ddATP, ddCTP and ddGTP at different concentrations (33 µM, 66 µM and 166 µM). The average fragment length of the resulting cDNA depends on the concentration of the mixture of ddATP, ddCTP and ddGTP (Figure 2). Thus, cDNA average fragment length can be controlled by the ddNTP concentration. Only the cDNA is visible on the gel as the RT primer was labeled with a fluorescent dye.

### Example 2: Specific degradation of the primer by using Exonuclease T

The cDNA has been synthesized as described above. The mixture comprising the cDNA and the non-incorporated primer is exposed to Exonuclease T treatment. The Exonuclease T requires a free 3' terminus and removes nucleotides in the 3'→ 5' direction. Due to the incorporation of the ddNTPs, 3' terminus of the cDNA does not have a 3' hydroxyl group. Therefore, only the primer but not the cDNA is degraded by Exonuclease T (Figure 3). Only the cDNA is visible on the gel as the RT primer was labeled with a fluorescent dye.

### Example 3: Circularization of 3' ddNTP-blocked ssDNA

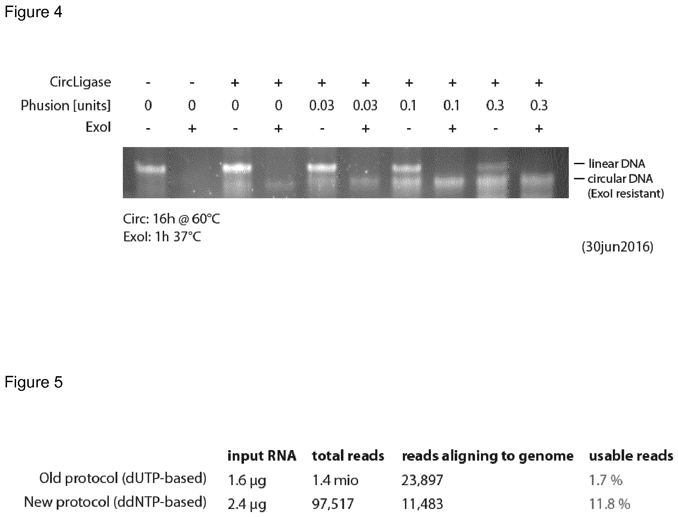

cDNA synthesized as described above is combined with CircLigase and Phusion polymerase. The Phusion polymerase is thermo-stabe and possesses 3'→ 5' exonuclease activity to cleave the ddNTPs from the 3' terminus of the cDNA. This cleaved cDNA can subsequently be circularized by CircLigase (Figure 4).

### Example 4: Comparison between of the method for gene analysis of the present invention and the method disclosed in European patent application EP3015554

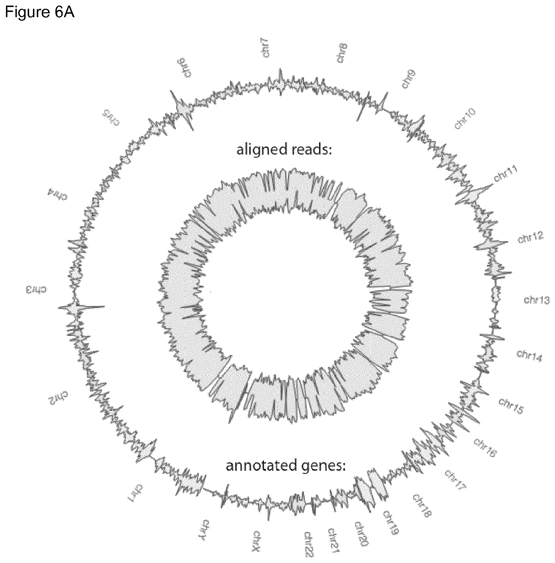

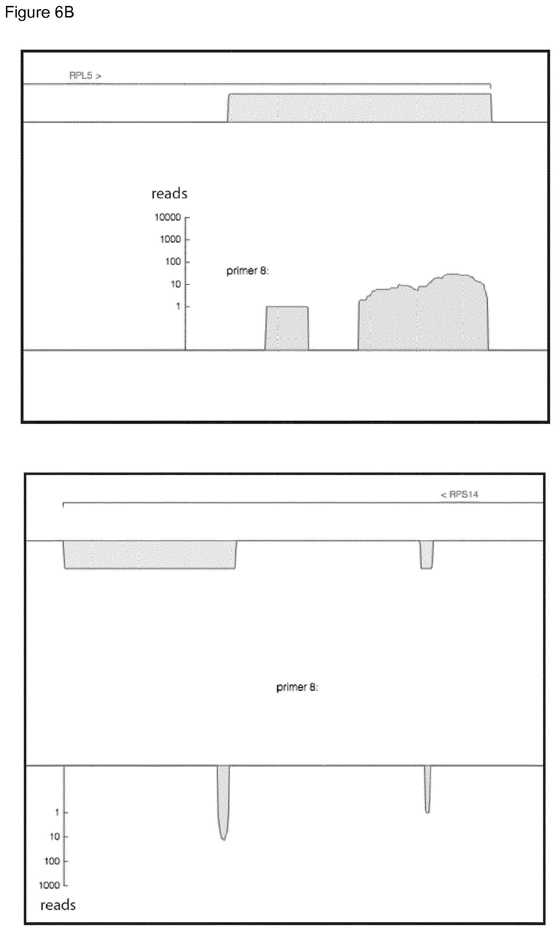

The percentage of meaningful reads (reads aligning to the genome) from cDNA, which were synthesized by the method of the present invention or the method of EP3015554, were compared after sequencing. The method of EP3015554 yielded 1.7% usable reads, whereas the present method provides almost seven-times more usable reads, namely 11.8%. Thus, the method of the present invention compares favorably over former protocol of EP3015554 (Figures 5 and 6).

## Claims

1. A method for gene analysis, preferably gene expression profiling comprising:
a) providing nucleic acid;
b) transcribing the nucleic acid to form single-stranded DNA having at its 3'-end a ddATP, ddCTP, ddGTP, or ddTTP nucleotide or a dATP, dCTP, dGTP, or dTTP nucleotide comprising a modified 3'-moiety by contacting the nucleic acid with a DNA polymerase, a primer and a mixture of (a) dNTPs and (b) at least one ddNTP or at least one dNTP comprising the modified 3'-moiety under conditions that allow the generation of the DNA, wherein the primer comprises a target-complementary region and optionally wherein the amounts of dNTPs are essentially equimolar;
c) degrading the non-elongated primer;
d) optionally purifying the DNA;
e) contacting the DNA with a 3' to 5' exonuclease to remove the ddATP, ddCTP, ddGTP or ddTTP nucleotide or the dATP, dCTP, dGTP, or dTTP nucleotide comprising the modified 3'-moiety;
f)i) contacting the DNA with a ssDNA ligase to circularize the DNA; or
f)ii) contacting the DNA with
(1) a linker molecule comprising a nucleic acid portion; and
(2) a DNA ligase to ligate the DNA to the linker molecule;
g) optionally amplifying the DNA;
h) optionally quantifying the DNA by qPCR;
i) sequencing the DNA.

2. The method of claim 1, wherein the nucleic acid of step a) is provided in form of a sample and is selected from genomic DNA and or cellular RNA.

3. The method of claim 2, wherein the RNA is mRNA, preferably eukaryotic mRNA.

4. The method of claim 3, wherein the eukaryotic mRNA comprises a plurality of different mRNAs, preferably comprises the complete cellular mRNA population.

5. The method of claim 3 or 4, wherein
(i) in step b) the mRNA is reverse transcribed to form single-stranded cDNA by contacting the mRNA with a RNA-dependent DNA polymerase, preferably reverse transcriptase (RT), a universal RT primer and a mixture of (a) dNTPs and (b) at least one ddNTP or at least one dNTP comprising the modified 3'-moiety under conditions that allow the generation of the cDNA, wherein the universal RT primer comprises at its 3'-end a target-complementary region comprising or consisting of a poly(dT)-3'OH or poly(dT)-dV-3'OH or poly(dT)-dV-dN-3'OH sequence to facilitate hybridization to the polyadenylation sequence of the mRNA, wherein the poly(dT) sequence is 5 to 50 nucleotides in length and V is A, C or G and N is A, C, G or T; and/or
(ii) optionally, the method additionally comprises the step of digesting the mRNA by contacting it with an RNAse at any time prior to step e).

6. The method of any one of claims 1 to 5, wherein the primer comprises a 3' target-complementary region and at its 5'-end any one or more target non-complementary regions selected from the group consisting of: a unique identifier (barcode) sequence, a sequencing primer binding site and optionally one or more sequencing adapter sites, a 5' mono-phosphorylation or 5'-adenylation, a detectable label, preferably a fluorescent label, and a non-base spacer; preferably at least a unique identifier sequence and a sequencing primer binding site.

7. The method of any of claims 1 to 6, wherein the primer is an universal RT primer, preferably 5'-mono-phosphorylated, and comprises in 3' to 5' orientation either (a) a target-complementary region, preferably a poly(dT) sequence, a sequencing adapter, optionally a blocking spacer, optionally a detectable label, optionally a second sequencing adapter, a sequencing primer binding-site, and an identifier barcode sequence or (b) a target-complementary region, preferably a poly(dT) sequence, an identifier barcode sequence, a sequencing primer binding-site, a sequencing adapter, optionally a blocking spacer, optionally a detectable label, and optionally a second sequencing adapter.

8. The method of any one of claims 1 to 7, wherein
(a) the ratio between the ddNTPs or the dNTP nucleotides comprising a modified 3'-moiety and the respective dNTPs is in the range of 1:1 to 1:1000, preferably 1:5 to 1:20;
(b) the ddNTP nucleotides consist of ddATP, ddCTP, ddGTP and any combination thereof, preferably ddVTPs; or
(c) the dNTP nucleotides comprising a modified 3'-moiety consist of modified dATP, dCTP, dGTP and any combination thereof, preferably modified dVTPs.

9. The method of any one of claims 1 to 8, wherein
(a) the method further comprises the step of quantifying the circularized DNA prior to the sequencing step e); and/or
(b) steps e) and f)i) or steps e) and f)ii) are accomplished at the same time.

10. The method of any one of claims 1 to 9, wherein the step c) of degrading the non-elongated primer comprises an enzymatic digestion, preferably digestion by an exonuclease, more preferably by Exonuclease T (Exo T), T4 DNA polymrease, Klenow fragment or Exonuclease I, most preferably by Exonuclease T.

11. The method of any one of claims 1 to 10, wherein steps a) and b) are performed for a plurality of different nucleic acid or mRNA populations, wherein for each population a universal (RT) primer with a different unique identifier sequence is used, wherein the thus generated plurality of DNA molecules is then combined for all subsequent method steps.

12. The method of any one of claims 1 to 11, wherein step d) comprises precipitation, chromatography, column purification, and/or gel electrophoresis.

13. The method of any one of claims 1 to 12, wherein step a) comprises isolating the nucleic acid from a sample.

14. A kit for gene analysis, preferably gene expression profiling, comprising
(A) DNA polymerase;
(B) one or more primers comprising a target-complementary region;
(C) a composition comprising dNTPs;
(D) a composition comprising ddATP, ddCTP, ddGTP, ddTTP or a dATP, dCTP, dGTP, or dTTP nucleotide comprising a modified 3'-moiety or any combination thereof, optionally in form of a mixture with the composition of (C);
(E) 3' to 5' exonuclease not catalyzing the digestion of a nucleic acid having at its 3' terminal end a ddATP, ddCTP, ddGTP, ddTTP, or a nucleotide of dATP, dCTP, dGTP, or dTTP comprising a modified 3'-moiety, preferably Exonuclease T and/or Exonuclease I;
(F) ssDNA ligase; and
(G) 3' to 5' exonuclease catalyzing the digestion of a nucleic acid having at its 3' terminal end a ddATP, ddCTP, ddGTP, ddTTP, or a nucleotide of dATP, dCTP, dGTP, or dTTP comprising a modified 3'-moiety.

15. The kit of claim 14, further comprising:
(H) sequencing primers.


SEQUENCE LISTING

<110>  Rheinische Friedrich-Wilhelms-Universität Bonn

<120>  Gene Expression Analysis

<130>  P66544

<160>  5

<170>  PatentIn version 3.5

<210>  1
<211>  100
<212>  DNA
<213>  Artificial Sequence

<220>
<223>  Primer

<400>  1
gatcagatcg gaagagcgtc gtgtagggaa agagtgtaga tctcggtggt cgccgtatca      60

agcagaagac ggcatacgag attttttttt tttttttttt                            100


<210>  2
<211>  100
<212>  DNA
<213>  Artificial Sequence

<220>
<223>  Primer

<400>  2
gacgtagatc ggaagagcgt cgtgtaggga aagagtgtag atctcggtgg tcgccgtatc      60

aagcagaaga cggcatacga gatttttttt tttttttttv                            100


<210>  3
<211>  23
<212>  DNA
<213>  Artificial Sequence

<220>
<223>  SBS3 fwd

<400>  3
agatcggaag agcgtcgtgt agg                                               23


<210>  4
<211>  23
<212>  DNA
<213>  Artificial Sequence

<220>
<223>  circ qPCR rev

<400>  4
aatgatacgg cgaccaccga gat                                               23

<210>  5
<211>  100
<212>  DNA
<213>  Artificial Sequence

<220>
<223>  CircMrna 8

<400>  5
ggtgaagatc ggaagagcgt cgtgtaggga aagagtgtag atctcggtgg tcgccgtatc      60

aagcagaaga cggcatacga gatttttttt tttttttttv                              100